# EUROPEAN PATENT SPECIFICATION

(11) **EP 1 199 301 B1**
(45) Date of publication and mention of the grant of the patent: **08.06.2005**
(21) Application number: 01308423.1
(22) Date of filing: 02.10.2001
(51) Int. Cl.: C07C 67/30, C07C 69/24

(54) **Olefination process to itaconate and succinate derivatives**
Olefinierungsverfahren zur Herstellung Itaconsäure- und Bernsteinsäurederivaten
Formation d'oléfines pour la préparation de dérivés d'acide itaconique et succinique

(30) Priority: 16.10.2000 GB 0025310
(43) Date of publication of application: 24.04.2002
(73) Proprietor: Pfizer Limited, Sandwich, Kent CT13 9NJ (GB); PFIZER INC., New York, N.Y. 10017 (US)
(72) Inventor: Derrick, Andrew Michael, Sandwich, Kent CT13 9NJ (GB); Thomson, Nicholas Murray, Sandwich, Kent CT13 9NJ (GB)
(74) Representative: Motion, Keith Robert

(56) References cited:
- EP-A- 0 561 758
- WO-A-01/47901
- WO-A-95/04033
- WO-A-99/35124
- W. M. OWTON ET AL.: "Tert-butyl 3-carboxyethyl-3-phosphonodiethylpropionat e. A novel reagent for Stobbe-like condensation" SYNTHETIC COMMUNICATION, vol. 23, no. 15, 1993, pages 2119-25, XP001055626
- FRAY M J ET AL: "Discovery of potent and selective succinyl hydroxamate inhibitors of matrix metalloprotease-3 (Stromelysin-1)" BIOORGANIC & MEDICINAL CHEMISTRY LETTERS, OXFORD, GB, vol. 11, no. 4, 26 February 2001 (2001-02-26), pages 571-574, XP004230062 ISSN: 0960-894X

## Description

The invention described herein relates to an olefination process which is useful for making certain itaconate and succinate derivatives.

It is desirable in a number of instances to be able to have an efficient and selective process, capable of scale-up, to make itaconate derivatives of formula (I), and/or succinate derivatives of formula (II) and/or (III): 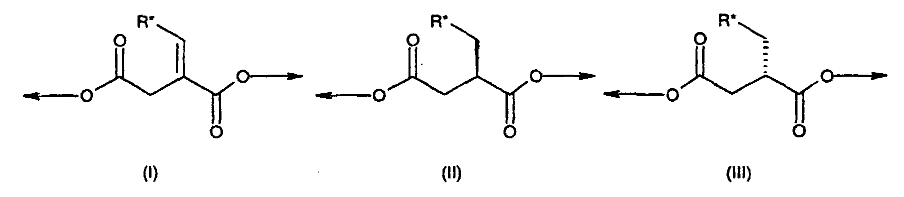 wherein "R*" is a sterically bulky group.

Of particular interest to us is the provision of compounds of the formula (IV), (V) and (VI), especially (IV) and (V): 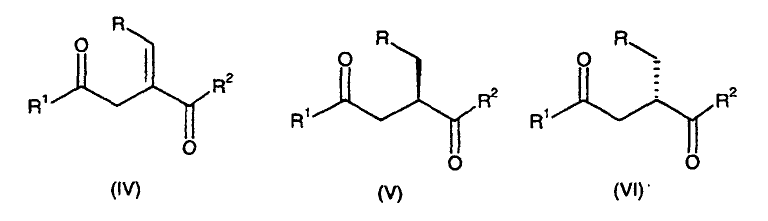 wherein R is aryl, C₃₋₈ cycloalkyl, C₁₋₁₀ alkyl, (aryl)C₁₋₁₀ alkylene, (C₃₋₈ cycloalkyl)C₁₋₁₀ alkylene, heterocyclyl, (heterocyclyl)C₁₋₁₀ alkylene, (aryl)C₃₋₈ cycloalkylene, (C₃₋₈ cycloalkyl)arylene or (C₁₋₁₀ alkylaryl)C₁₋₁₀ alkylene, wherein "aryl" is a mono- or bicyclic partially or fully unsaturated carbocyclic ring system containing from 4 to 10 atoms, such as phenyl or naphthyl, or a partially or fully unsaturated mono- or bicyclic heterocyclic moiety having up to 10 atoms in the ring system and with up to 4 hetero-atoms in the said ring system each independently selected from N, O and S,
said carbocyclic ring system and heterocyclic moiety being optionally substituted by one or more substituents each independently selected from halogen, NO₂, NH₂, CO₂R⁹, phenyl, C₁₋₆ alkyl(optionally substituted by one or more halogen), and C₁₋₆ alkoxy(optionally substituted by one or more halogen), and
"heterocyclyl" is a 3- to 8-membered mono or bicyclic saturated heterocyclic group having from 1 to 4 ring hetero-atoms each independently selected from N, O and S, optionally substituted by one or more substituents each independently selected from halogen, NO₂, NH₂, CO₂R⁹, phenyl, C₁₋₆ alkyl(optionally substituted by one or more halogen), and C₁₋₆ alkoxy(optionally substituted by one or more halogen);
R¹ is C₁₋₆ alkoxy,
R² is OH or O⁻M⁺;
R⁹ is H or C₁₋₆ alkyl;
and M⁺ is the cation of a metal such as sodium, lithium or potassium, or is a protonated amine moiety such as (mono-, di- or tri-C₁₋₁₀ alkyl)ammonium, (mono-, di- or tri-C₃₋₁₀ cycloalkyl)ammonium, (C₁₋₁₀ alkyl)ₙ₁(C₃₋₁₀ cycloalkyl)ₙ₂ammonium, anilinium, benzylammonium, triethanolammonium, or (*S*)-α-methylbenzylammonium, where
n1 and n2 are each independently selected from 1 or 2 with the proviso that the sum of n1 and n2 is not greater than 3;
Alkyl groups, and groups containing alkyl moieties such as alkoxy and alkylene groups, can be straight chain or branched if the number of carbon atoms allows,
Halogen means fluorine, chlorine or bromine,
Cycloalkyl groups attached to an ammonium moiety can contain 1, 2, or 3 rings, where the number of carbon atoms allows, for example adamantanammonium.

Production of compounds related to (IV), (V) and (VI) has been disclosed previously, e.g. by Owton et al, in Synthetic Communications, 23(15), 2119-2125 (1993), MJ Burk et al, Angew. Chem. Int. Edn. (Eng.) (1998) 37, 13/14, 1931-1933, Monsanto, US Patent 4,939,288 or EP Patent 561758 by Chirotech Technology Ltd. in International Patent Application publication no. WO 99/31041. Known olefination reactions leading to systems related to (IV), generally result in poor E/Z selectivity, (for a review of the Stobbe condensation, see Org.React. 1951, 6, 1-73). Where selectivity has been controlled, however, for example by the use of phosphorus reagents, the substitution pattern is different from that required by us in formula (IV)(e.g. Monsanto, Owten, *supra*).

The products of the Owten chemistry are exemplified by compounds of the formula (VII): 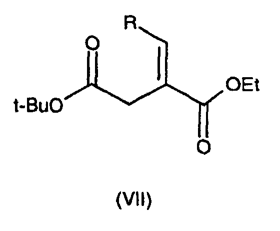 on which we attempted hydrolysis of the ethyl ester using conventional chemistry. In our hands this resulted in scrambling of the olefinic moiety resulting overall in a mixture of stereoisomers and regioisomers.

Use of the Monsanto chemistry gives products of the formula (VIII): 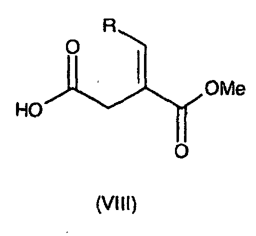 which has the wrong substitution pattern for our requirements.

We have discovered an efficient olefination method which can be used to make compounds of formula (IV) in good yield and with good trans-selectivity, and which products can then be asymmetrically reduced to give compounds of formula (V) and (VI). The olefination is base-catalysed and can be used with enolisable aldehydes or aldehyde derivatives without significant amounts of self-condensation products. We also surprisingly observe no substantial double bond migration to give deconjugated isomers of (IV) under the basic conditions, which would afford other geometric and regio-isomers, which is another significant problem with similar prior art olefinations.

Our olefination system is thus particularly useful when highly selective production of the compounds (IV), (V) and/or (VI) is required, or where separation of (IV), (V) and/or (VI) and/or the respective isomers thereof, may be difficult or undesirable, such as in processing to make pharmaceutical products and regulatory starting materials for such products.

Thus, according to the present invention, there is provided a process for the preparation of compounds of formula (IV) as defined above, comprising reaction of an aldehyde of formula RCHO, or a protected derivative thereof such as a hemiacetal or adduct thereof such as a bisulphite, wherein R is as defined above,
with a phosphorus compound of formula (IX): 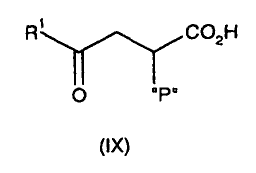 or a metal carboxylate salt thereof such as a sodium, lithium or potassium carboxylate salt thereof,
wherein R¹ is as defined above, and
"P" is a phosphonate moiety of formula -P(O)(OR³)(OR⁴), wherein R³ and R⁴ are either each independently selected from H, C₁₋₆ alkyl, benzyl and phenyl (optionally substituted by one or more C₁₋₆ alkyl), or R³ and R⁴ taken together are C₂₋₅ alkylene,
or "P" is a phosphorane moiety of formula -(PR⁵R⁶R⁷)⁺X⁻ wherein R⁵, R⁶ and R⁷ are each independently selected from C₁₋₆ alkyl and phenyl, and X is bromine, chlorine or iodine,
in the presence of a sodium, lithium or potassium C₁-C₆ alkoxide base,
in an inert solvent, and
at a temperature of from -80°C to 20°C.

Preferably the reaction time is less than 24 hours.

Preferably R is (aryl)C₁₋₁₀ alkylene or (C₃₋₈ cycloalkyl)C₁₋₁₀ alkylene.
More preferably R is phenylethyl, cyclohexylethyl or (2-methyl-1,1'-biphenyl-4-yl)ethyl.

Preferably R¹ is t-butoxy.

Preferably R² is OH, O⁻Li⁺, O⁻Na⁺, O⁻K⁺, O⁻cyclohexylammonium⁺, O⁻ adamantanammonium⁺, O⁻triethanolammonium⁺ or O⁻(*S*)-α-methylbenzylammonium⁺.

Preferably the olefination reaction is carried out using the aldehyde RCHO or the sodium bisulphite adduct thereof RCH(OH)SO₃⁻Na⁺.

Preferably "P" is P(O)(OC₂H₅)₂, P(O)(OCH₂CH₂O) or a triphenylphosphinium halide moiety.
More preferably P is P(O)(OC₂H₅)₂.

Preferably the base is potassium t-butoxide, sodium t-butoxide or sodium methoxide.
When the the base alkoxide and R¹ are different there is a possibility of transesterification taking place during the olefination reaction. We have found that this apparently has no detrimental effect on the course of the reaction at the olefination centre, in terms of stereochemistry, and may not be important with respect to the use made of the product, e.g. if it is used as an intermediate and the R¹ moiety is later removed, e.g. by displacement, hydrolysis or deprotection.

Preferably the olefination reaction solvent is anhydrous tetrahydrofuran, anhydrous toluene or R¹H, where R¹ takes the meaning as specified above with respect to the compounds of formulae (IV), (V) and (VI), or a mixture thereof.
More preferably the reaction solvent is selected from tetrahydrofuran and toluene when the aldehyde RCHO is used as a substrate, and selected from tetrahydrofuran/t-butanol and toluene when the bisulphite adduct is used as substrate.

Preferably the reaction is carried out at a temperature from -20°C to 10°C. More preferably the reaction is carried out at a temperature from -10°C to 10°C.
Most preferably the reaction is carried out at a temperature from 0°C to 5°C.

When the aldehyde RCHO is used as substrate, it is preferable to add the phosphorus compound to the base/solvent mixture, followed by addition of the aldehyde. Alternatively, the phosphorus compound and aldehyde are combined, then the base is added. A further alternative is to add the base to the phosphorus compound followed by addition of the aldehyde.

When the bisulphite is used as the substrate, it is preferable to add the base to a mixture of the bisulphite adduct and the phosphorus compound, or, alternatively, the bisulphite is added to a mixture of the phosphorus compound and the base.

'Bisulphite' or 'bisulphite adduct' is taken to mean an α-hydroxysulphonate, which is the product of the reaction of an aldehyde with sodium, potassium or lithium bisulphite. Other suitable bisulphites are known in the art.

The skilled person will appreciate that the substrates, and starting material of formula (IX), can be obtained from commercial sources, or made by methods known in the art, e.g. by adaptation of the methods herein described in the sections below, and/or adaptation thereof, for example by methods known in the art. Suitable guides to synthesis, functional group transformations, use of protecting groups, etc. are found in standard organic chemistry textbooks, for example, "Comprehensive Organic Transformations" by RC Larock, VCH Publishers Inc. (1989), "Advanced Organic Chemistry" by J March, Wiley Interscience (1985), "Designing Organic Synthesis" by S Warren, Wiley Interscience (1978), "Organic Synthesis - The Disconnection Approach" by S Warren, Wiley Interscience (1982), "Guidebook to Organic Synthesis" by RK Mackie and DM Smith, Longman (1982), "Protective Groups in Organic Synthesis" by TW Greene and PGM Wuts, John Wiley and Sons Inc. (1999), and PJ Kocienski, in "Protecting Groups", Georg Thieme Verlag (1994), and any updated versions of said standard works.

The above olefination process is optionally followed by conversion of the product of formula (IV) where R² is O⁻M⁺ wherein M⁺ is a metal such as Na, Li or K, to a compound of formula (IV) where R² is OH by treatment with a proton source, which may optionally be converted to a compound of formula (IV) wherein R² is O⁻M⁺(where M⁺ is a protonated amine moiety as previously defined), by treatment with a corresponding amine.

A further aspect of the invention is the asymmetric hydrogenation of itaconate compounds of the formula (IV) to give succinate compounds of the formula (V) or (VI).

Asymmetric hydrogenation of compounds of formula (IV) may be achieved in a multitude of ways, including methods known in the art. For example use may be made of catalytic hydrogenation using chiral rhodium or ruthenium complex of an optically active biphosphine or biphosphinite compound such as (4R,5R)-(-)-O-Isopropylidene-2,3-dihydroxy-1,4-bis(diphenylphosphino)butane [(R,R)-DIOP], (R,R)-(-)-1,2-Bis[(O-methoxyphenyl)(phenyl)phosphino]ethane [(R,R)-DIPAMP], (-)-(R)-N,N-Dimethyl-1-((S)-1',2-Bis(Diphenylphosphino)ferrocenyl)ethylamine [(R)-(S)-BPPFA], (-)-(2S,4S)-2-Diphenylphosphinomethyl-4-diphenylphosphino-1-t-butoxycarbonylpyrrolidine [(S,S)-BPPM], (2S,3S)-(-)-Bis(diphenylphosphino)butane [(S,S)-CHIRAPHOS], R-(+)-1,2-Bis(diphenylphosphino)propane [(R)-PROPHOS], (2R,3R)-(-)-2,3-Bis(diphenylphosphino)bicyclo[2.2.1]hept-5-ene [(R,R)-NORPHOS], (R)-(+)-2,2'-Bis(diphenylphosphino)-1,1'-binaphthyl [(R)-BINAP], (R)-1,2-bis(diphenylphosphino)-1-cyclohexylethane [(R)-CYCPHOS], (2R,4R)-(+)-2,4-Bis(diphenylphosphino)pentane [(R,R)-BDPP], N-benzyl-(3R,4R)-3,4-bis(diphenylphosphino)pyrrolidine [(R,R)-DEGPHOS], (-)-1,2-Bis((2R,5R)-2,5-dimethylphospholano)benzene [(R,R)-Me-DUPHOS], (-)-1,2-Bis((2R,5R)-2,5-diethylphospholano)benzene [(R,R)-Et-DUPHOS], N,N'-bis[(R)-(+)-α-methylbenzyl]-N,N'-bis(diphenylphosphino)ethylenediamine [(R)-PNNP], (R)-(-)-2,2'-bis(dicyclohexylphosphino)-6,6'-dimethyl-1,1'-biphenyl [(R)-BICHEP], (1R,2S,4R,5S)-2,5-dimethyl-7-phosphadicyclo[2.2.1]heptane [(R,S,R,S)-Me-PENNPHOS], (2S,2'S)-Bis(diphenylphosphino)-(1S,1'S)-bicyclopentane [(S,S)-BICP], 1,1'-bis[(2*S*,4*S*)-2,4-diethylphosphetano]ferrocene [(S,S)-Et-FerroTANE], (R,R)-1,2-bis(di-t-butylmethylphosphino)methane [(R,R)-t-butylminiPHOS], (R)-(+)-2,2'-Bis(di-p-tolylphosphino)-1,1'-binaphthyl [(R)-tol-BINAP], (R)-(+)-2-(Diphenylphosphino)-2'-methoxy-1,1'-binaphthyl [(R)-MOP], (R)-(+)-1-(2-Diphenylphosphino-1-naphthyl)isoquinoline [(R)-QUINAP], Methyl α-D-glucopyranoside-2,6-dibenzoate-3,4-di(bis(3,5-dimethylphenyl)phosphinite) (CARBOPHOS), (R)-(-)-1-[(S)-2-(Diphenylphosphino)ferrocenyl]ethyldicyclohexylphosphine [(R)-(S)-JOSIPHOS], (R)-(-)-4,12-Bis(diphenylphosphino)-[2.2]-paracyclophane [(R)-PHANEPHOS], (R)-(6,6'-Dimethoxybiphenyl-2,2-diyl)-bis(diphenylphosphine) [(R)-MeO-BIPHEP],], (R)-(6-Chloro-6'-methoxybiphenyl-2,2-diyl)-bis(diphenylphosphine) [(R)-Cl-MeO-BIPHEP] or 2,2'-bis(diphenylphosphino)-4,4',6,6'-tetrakis(trifluoromethyl)-1,1'-biphenyl (BIFUP) which are well-known to the skilled chemist working in the asymmetric hydrogenation art.

It is to be appreciated that other examples include related structures, such as those with alternative alkyl substituents, and stereoisomers of the above-mentioned examples.

Some catalysts are mentioned in the Examples, and other suitable catalysts which may be useful are mentioned in the following publications and references therein, all of which are herein incorporated by reference in their entirety:
US Patent 4,939,288 (Monsanto);
International Patent Application publication no. WO 98/02445 (Chiroscience Ltd);
International Patent Application publication no. WO 99/31041 (Chirotech Technology Ltd);
European Patent Application 0 673 911 A1 (Takasago International Corporation);
International Patent Application publication no. WO 00/27855 (Chiroscience Technology Ltd);
X. Zhang, Enantiomer (1999), 4(6), 541;
H.Tye, JCS Perkin I, (2000) (3) 275-298;
J.M. Brown, Compr. Asymmetric Catal. I-III (1999), 1, 121-182;
M.J. Burk, Spec.Chem. (1998) 18(2) 58-59, 62;
T. Yamagishi, Organomet. News (1995) (4), 113-118;
J.P. Genet, ACS Symp. Ser. (1996) 641 (Reductions in Organic Synthesis), 31-51;
H. Kumobayashi, Recl.Trav.Chim.Pays-Bas (1996) 115(4) 201-210;
M.J. Burk et al, Pure Appl. Chem. (1996) 68(1) 37-44;
H. Takaya et al, Catal. Asymm.Synth. (1993) 1-39;
S.Akutagawa, Chirality Ind. (1992) 325-339; A.Borner et al, Transition Met.Org.Synth. (1998) 2 3-13;
D.G.Blackmond, CATTECH (1998) 2(1), 17-32;
R.Noyori, Acc.Chem.Res. (1997) 30(2) 97-102;
W.S.Knowles, Chem.lnd.(Dekker) (1996) 68(Catalysis of Organic Reactions) 141-152;
U.Behrens, Spec.Chem.(1996) 16(5) 174, 176-177;
R.Noyori, Acta Chem.Scand. (1996) 50(4), 380-390;
H.B.Kagan, Mec., Phys., Chim., Astron., (1996) 322(2) 131-143;
A.S.C.Chan et al, Chem.lnd.(Dekker) (1994) 53(Catalysis of Organic Reactions) 49-68;
K.Inoguchi et al, Synlett (1992) (3) 169-78;
G.Webb et al, Catal.Today (1992) 12(2-3) 319-337;
D.Arntz et al; Catal.Met.Complexes (1991) 12(Met.Promoted Sel.Org.Synth.) 161-189;
K.Harada, Asymmetric Synth. (1985) 5 345-383; and
W.S.Knowles, Acc. Chem.Res.(1983) 16(3) 106-112.

The person skilled in the art will appreciate that the use of one enantiomer of such chiral catalysts will give one enantiomer (V) or (VI), and use of the other enantiomer will give the other enantiomer.

Some of the suitable catalysts may be generically defined by the formula P*-cat-P** wherein "cat" is a metal such as rhodium or ruthenium, and P* and P** each independently represents a chiral phosphine moiety, optionally conjoined.

Preferably the catalyst used for reduction of compounds of formula (IV) where R² is O⁻M⁺, is ruthenium based, such as ruthenium complexes of BINAP and derivatives thereof, such as [(*S*)-2,2'-bis(diphenylphosphino-1,1'-binaphthyl]chloro(*p*-cymene)ruthenium chloride.

Preferably the catalyst used for reduction of compounds of formula (IV) where R² is OH, is rhodium-based, such as Rh-DUPHOS (1,2-bis[(2S,5S)-2,5-diethylphospholano]benzene-(1,5-cyclooctadien)-rhodium (I) tetrafluoroborate) or Rh-Ferrotane (1,1'-bis[(2S,4S)-2,4-diethylphosphetano]ferrocene-(1,5-cyclooctadiene)-rhodium (I) tetrafluoroborate).

Suitably the hydrogenation of the acid (IV, R² is OH), can be carried out in the presence of a base such as sodium bicarbonate, cyclohexylamine, isopropylamine, t-butylamine, adamantanamine, or (S)-α-methylbenzylamine. The hydrogenation can be carried out on a preformed salt (IV, R² is O⁻M⁺).

The reaction is suitably carried out in an inert solvent such as an aqueous C₁₋₃ alcohol e.g. aqueous methanol, or C₁₋₃ alcohol e.g. methanol. Other suitable inert solvents include, but is not limited to, tetrahydrofuran, ethyl acetate, *tert*-butyl methyl ether, α, α, α- trifluorotoluene, methylene chloride or toluene. The reaction is carried out optionally at an elevated temperature, under a positive pressure of hydrogen.

Suitable temperatures for good yield and selectivity for the ruthenium-catalysed hydrogenation has been found to be approximately 60°C, and for the rhodium-catalysed reactions at approximately 20°C.

The skilled chemist will realise that suitable conditions for particular hydrogenations of compounds of formula (IV) can be found by routine modification of those mentioned herein.

The invention is illustrated in the following Examples and Preparations section, but is not limited to these illustrations.

### Preparation 1: 3-(diethoxyphosphoryl)succinic acid 1-tert-butyl ester

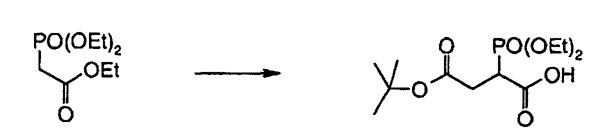

Triethylphosphonoacetate (12.0Kg, 53.5 mol) was added over 30 minutes to a stirred solution of potassium *tert*-butoxide (7.20Kg, 64.2 mol) in THF (118 litres), between 0 and 5°C, under nitrogen. The mixture was warmed to 25-30°C where it was stirred for 1 hour and then added over 45 minutes to a solution of *tert*-butyl bromoacetate (11.5Kg, 59.0 mol) in THF (28 litres), between 0 and 5°C, under nitrogen. The mixture was stirred at 0-5°C for 1 hour and then demineralised water (6.1 litres) and ethanol (30 litres) were added. A solution of potassium hydroxide (4.2Kg, 75.0 mol) in demineralised water (84 litres) was then added over 2 hours, between -5 and 0°C. The mixture was stirred at -10°C for 16 hours and then a solution of citric acid (16.5Kg, 85.8 mol) in demineralised water (32 litres) was added. The mixture was concentrated *in vacuo* to a volume of 180 litres and then ethyl acetate (90 litres) was added. The organic phase was separated and the aqueous phase was re-extracted with ethyl acetate (30 litres). The combined organic phases were washed with water (30 litres) and then stripped and replaced with cyclohexane by distillation at atmospheric pressure, at a constant volume of 72 litres. *tert*-Butylmethyl ether (18 litres) was added and the mixture was stirred at 20-25°C for 12 hours and then filtered. The residue was washed with a mixture of cyclohexane (16 litres) and *tert*-butylmethyl ether (3.6 litres) then dried *in vacuo* for 16 hours to give the title compound as a colourless solid (10.0Kg, 60% yield, 98% pure by HPLC).
¹H-NMR (CDCl₃) δ : 4.20-4.10 (4H, m), 3.49-3.36 (1H, m), 3.00-2.85 (1 H, m), 2.72-2.60 (1H, m), 1.20 (9H, s), 1.37-1.27 (6H, m)

### Example 1: (E)-2-[2-(tert-butoxy)-2-oxoethyl]-5-phenyl-2-pentenoic acid

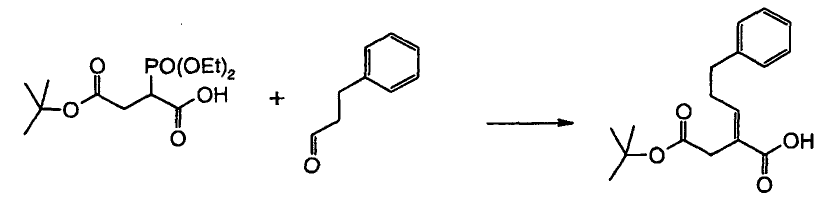

A solution of 3-(diethoxyphosphoryl)succinic acid 1-*tert-*butyl ester (100g, 0.32mol) in THF (300ml) was added dropwise over 15 min to a stirred solution of potassium *tert*-butoxide (110g, 0.98mol) in THF (300ml), between -10 and -5°C, under nitrogen. The mixture was stirred at -10°C for 15 min and then a solution of hydrocinnamaldehyde (46.8g, 0.35mmol) in THF (100ml) was added dropwise over 15 min, between -13 and -8°C. The mixture was stirred at -10°C for 30 min and then a solution of citric acid (111 g, 0.58mol) in demineralised water (500ml), and ethyl acetate (500ml), were added. The pH was adjusted to pH 4 with aqueous sodium hydroxide solution (50%) and the phases were separated. The aqueous fraction was washed with ethyl acetate (500ml) and the combined organic fractions were washed with saturated sodium bicarbonate solution (500ml), citric acid solution (10%, 500ml) and demineralised water (500ml) and then concentrated *in vacuo.* The resulting solid was slurried in cyclohexane (470ml) for 1 hour and then the mixture was filtered. The residue was washed with cyclohexane (2x50ml) and dried *in vacuo* to leave the title compound as a colourless solid (76g, 81 % yield, 99% pure by HPLC).
MS : 289 [(M-H)]⁻
¹H-NMR (CDCl₃) δ : 7.33-7.16 (5H, m), 7.05 (1H, br t), 3.20 (2H, s), 2.89 (2H, br t), 2.50 (2H, br dd), 1.41 (9H, s)

### Example 2: (R)-2-[2-(tert-butoxy)-2-oxoethyl]-5-phenylpentanoic acid

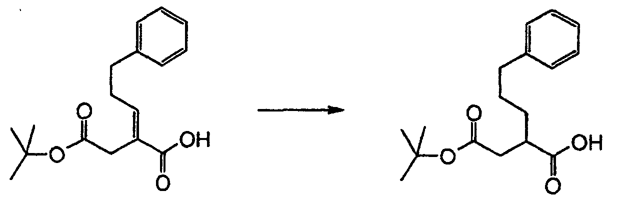

A solution of (*E*)-2-[2-(*tert*-butoxy)-2-oxoethyl]-5-phenyl-2-pentenoic acid (5.8g, 20 mmol) and 1,1'-bis[(2*S*,4*S*)-2,4-diethylphosphetano]ferrocene-(1,5-cyclooctadiene)-rhodium (I) tetrafluoroborate (7.4mg, 10µmol) in methanol (10ml) was stirred at 20-25°C for 24 hours, under hydrogen (4 atmospheres, 60p.s.i.). The mixture was then concentrated *in vacuo* to leave the title compound as a yellow oil (5.8g, 98% conversion, enantiomeric excess=97%, 95% pure by NMR).
¹H-NMR (CDCl₃) δ : 7.30-7.17 (5H, m), 2.85-2.78 (1H, m), 2.66-2.58 (3H, m), 2.37 (1 H, br dd), 1.75-1.51 (4H, m), 1.40 (9H, s)

### Example 3: (R)-2-[2-(tert-butoxy)-2-oxoethyl]-5-phenylpentanoic acid cyclohexylamine salt

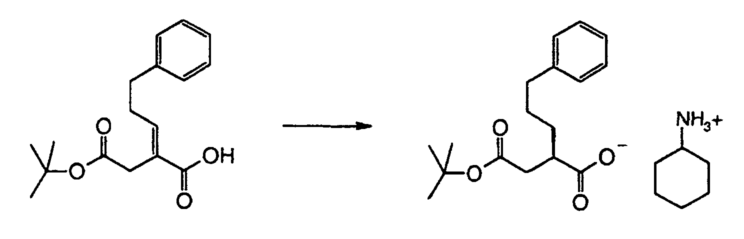

A stirred solution of cyclohexylamine (266ml, 2.32 mol), (*E*)-2-[2-(*tert*-butoxy)-2-oxoethyl]-5-phenyl-2-pentenoic acid (688g, 2.37 mol) and [(*S*)-2,2'-bis(diphenylphosphino-1,1'-binaphthyl]chloro(*p*-cymene)ruthenium chloride (4.4g, 4.7 mmol) in methanol (6.9 litres) was heated to 60°C, under hydrogen (60p.s.i.), for 47 hours and then allowed to cool to 20-25°C (enantiomeric excess= 88%). The mixture was filtered through a filter aid Celite® and then the solvent was stripped and replaced with acetone by distillation at atmospheric pressure, at a constant volume of 4.2 litres. The resulting suspension was cooled to 20-25°C, stirred for 4 hours and then filtered. The residue was washed with acetone (2x 1 litre) and then dried *in vacuo* at 45°C for 16 hours to leave the title compound as a colourless solid (590g, 64% yield, enantiomeric excess=98.9%, 97% pure by HPLC).
¹H-NMR (CD₃OD) δ: 7.23-7.09 (5H, m), 3.05-2.98 (1H, m), 2.64-2.56 (3H, m), 2.53 (1H, dd, *J* 15.2, 7.2Hz), 2.23 (1H, dd, *J* 15.2, 7.2Hz), 2.00-1.97, (2H, m), 1.85-1.81 (2H, m), 1.72-1.20 (10H, m), 1.40 (9H, s)

### Example 4: (R)-2-[2-(tert-butoxy)-2-oxoethyl]-5-phenylpentanoic acid sodium salt

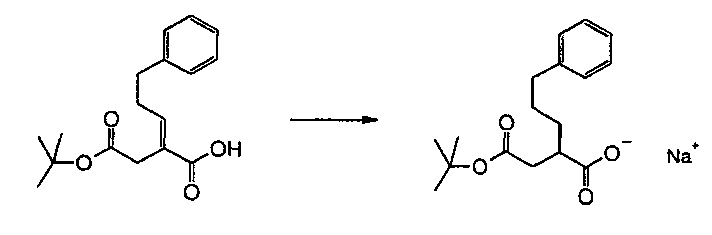

A stirred solution of sodium bicarbonate (28.6g, 0.34 mol), (*E*)-2-[2-(*tert*-butoxy)-2-oxoethyl]-5-phenyl-2-pentenoic acid (100g, 0.34 mol) and [(*S*)-2,2'-bis(diphenylphosphino-1,1'-binaphthyl]chloro(*p*-cymene)ruthenium chloride (0.63g, 0.68 mmol) in methanol (750ml) and water (250ml) was heated to 60°C, under hydrogen (60p.s.i.), for 24 hours and then allowed to cool to 20-25°C (enantiomeric excess 87%). The mixture was filtered through a filter aid Celite® and the solvent was stripped and replaced with acetonitrile by distillation at atmospheric pressure, at a constant volume of 500ml. The resulting suspension was cooled to 20-25°C and was stirred for 24 hours, then filtered. The residue was washed with acetonitrile (3x 25ml) and then dried *in vacuo* at 45°C for 3 hours to leave the title compound as a colourless solid (65g, 61% yield, enantiomeric excess=94.3%, 95% pure by NMR).
¹H-NMR (CD₃OD) δ : 7.23-7.10 (5H, m), 2.62-2.58 (3H, m), 2.53 (1 H, dd, *J* 15.2, 7.6Hz), 2.22 (1H, dd, *J* 15.2, 7.6Hz), 1.74-1.42 (4H, m), 1.40 (9H, s)

### Example 5: (E)-2-[2-(tert-butoxy)-2-oxoethyl]-5-cyclohexyl-2-pentenoic acid cyclohexylamine salt

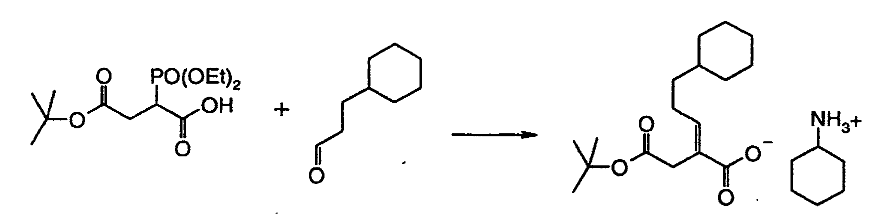

Potassium *tert*-butoxide (20.2g, 0.18mol) was added portionwise over 10 minutes to a solution of 3-(diethoxyphosphoryl)succinic acid 1-*tert*-butyl ester (25.4g, 82 mmol) in THF (115ml), at 0°C, under nitrogen. The mixture was stirred at 0°C for 40 minutes and then cooled to -20°C. A solution of 3-cyclohexylpropan-1-al (11.5g, 82 mmol in THF (60ml) was added dropwise over 10 minutes between -20 and -10°C, under nitrogen. The mixture was stirred between -20 and -5°C for 3 hours and then aqueous citric acid (10%, 250ml) and ethyl acetate (200ml) was added. The organic phase was separated and the aqueous fraction was washed with ethyl acetate (200ml).

The combined organic fractions were washed with saturated sodium bicarbonate solution (2x 100ml), aqueous citric acid solution (10%, 100ml) and demineralised water (100ml) and then concentrated *in vacuo.* The resulting solid was taken up in ethyl acetate (150ml) and cyclohexylamine (9.4ml, 82 mmol) was added dropwise over 5 minutes at 20-25°C. The mixture was stirred at 20-25°C for 16 hours and then filtered. The residue was dried *in vacuo* at 40°C for 4 hours to leave the title compound as a colourless solid (21.7g, 67% yield, 95% pure by NMR).
¹H-NMR (CD₃OD) δ : 6.70 (1H, t, *J* 7.2Hz), 3.26 (2H, s), 3.10-3.00 (1H, m), 2.76-2.63 (2H, m), 2.19-0.90 (23H, m), 1.41 (9H, s)

### Example 6: (R)-2-[2-(tert-butoxy)-2-oxoethyl]-5-cyclohexylpentanoic acid

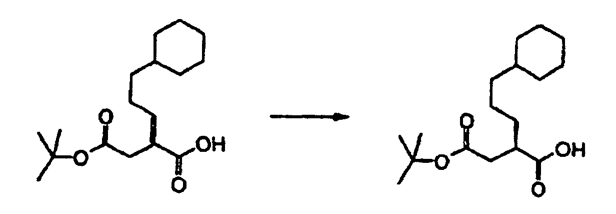

A solution of (*E*)-2-[2-(*tert*-butoxy)-2-oxoethyl]-5-cyclohexyl-2-pentenoic acid (0.74g, 2.5 mmol) and 1,1'-bis[(2*S*,4*S*)-2,4-diethylphosphetano]ferrocene-(1,5-cyclooctadiene)-rhodium (I) tetrafluoroborate (18mg, 25µmol) in methanol (2.5ml) was stirred at 20-25°C for 24 hours, under hydrogen (4 atmospheres, 60p.s.i.). The mixture was then concentrated *in vacuo* to leave the title compound as a yellow oil (0.74g, 98% conversion, enantiomeric excess=95%, 95% pure by NMR).
¹H-NMR (CDCl₃)δ : : 2.82-2.76(1H, m), 2.60(1H, br dd), 2.37(1H, br dd), 1.70-1.60 (6H, m), 1.51-1.30(3H, m), 1.42 (9H, s), 1.23-1.11 (6H, m), 0.96-0.80 (2H, m)

### Reference Example 7: (R)-2-[2-(tert-butoxy)-2-oxoethyl]-5-cyclohexylpentanoic acid

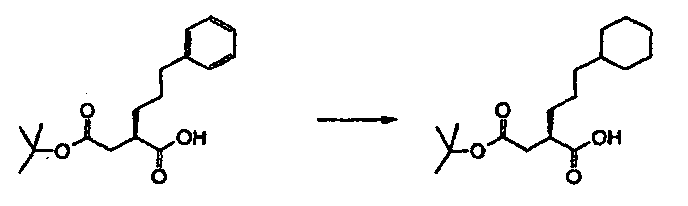

A solution of (*R*)-2-[2-(*tert*-butoxy)-2-oxoethyl]-5-phenylpentanoic acid (2.2g, 7.5mmol) and 5% rhodium on carbon (0.22g) in methanol (220ml) was stirred at 20-25°C, under hydrogen (10 atmospheres, 150p.s.i.) for 24 hours and then filtered through celite. The filtrate was concentrated *in vacuo* to leave the title compound as an oil (2.0g, 89% yield, 95% pure by NMR).
¹H-NMR (CDCl₃) δ : 2.82-2.76 (1H, m), 2.60 (1 H. br dd), 2.37 (1H, br dd), 1.70-1.60 (6H, m), 1.51-1.30 (3H, m), 1.42 (9H, s), 1.23-1.11 (6H, m), 0.96-0.80 (2H, m)

### Reference Example 8: (R)-2-[2-(tert-butoxy)-2-oxoethyl]-6-cyclohexylpentanoic acid cyclohexylamine salt

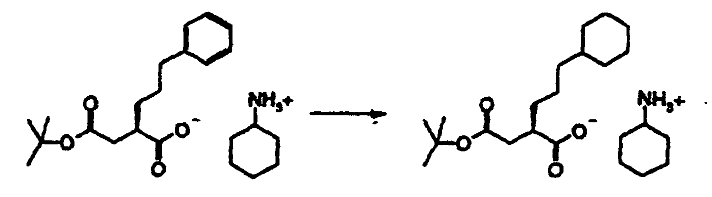

(*R*)-2-[2-(*tert*-Butoxy)-2-oxoethyl]-5-phenylpentanoic acid cyclohexylamine salt (691g, 1.77 mol) and ethyl acetate (7.0 litres) were added to an aqueous solution of citric acid (10%, 6.3 litres) and the organic phase was separated, washed with water (7.0 litres) and concentrated *in vacuo* to a yellow oil. A solution of the oil and 5% rhodium on carbon (51.6g) in methanol (7.0 litres) was stirred at 20-25°C, under hydrogen (10 atmospheres, 150p.s.i.) for 48 hours and then filtered through celite. To the filtrate was added cyclohexylamine (202ml, 1.77 mol) and the methanol solution was stripped and replaced with methylethyl ketone by distillation at atmospheric pressure, to a volume of 5.5 litres. The mixture was allowed to cool to 20-25°C where it was stirred for 48 hours and then filtered. The residue was washed with methylethyl ketone (2x 500ml) and then dried *in vacuo* at 45°C for 4 hours to leave the title compound as a colourless solid (495g, 71 %yield, 99% pure by HPLC).
¹H-NMR (CD₃OD) δ: 3.06-2.99 (1H, m), 2.63-2.56 (1H, m), 2.53 (1H, dd, *J* 15.2, 7.2Hz), 2.23 (1H, dd, *J* 15.2, 7.2Hz), 2.02-1.97 (2H, m), 1.77-1.15 (21H, m), 1.43 (9H, s), 0.93-0.82 (2H, m)

### Reference Example 9: (R)-2-[2-(tert-butoxy)-2-oxoethyl]-5-cyclohexylpentanoic acid sodium salt

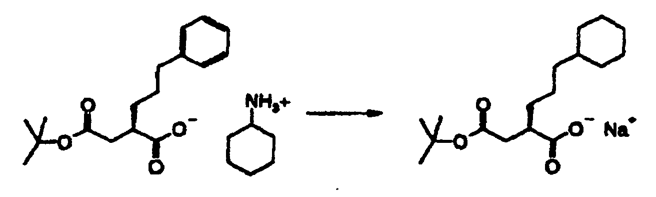

(*R*)-2-[2-(*tert*-Butoxy)-2-oxoethyl]-5-phenylpentanoic acid cyclohexylamine salt (6.8g,17 mmol) and ethyl acetate (100ml) were added to en aqueous solution of citric acid (10%, 100ml) and the organic phase was separated and washed with water (100ml). *Iso*-propyl alcohol (20ml) and 5% rhodium on alumina (51.6g) were added and the mixture was stirred at 20-25°C, under hydrogen (10 atmospheres,150p.s.i.) for 48 hours and then filtered through celite. To the filtrate was added a solution of sodium hydroxide (0.67g, 17 mmol) in water and the mixture was stripped and replaced with acetonitrile by distillation at atmospheric pressure to a volume of 30ml. The mixture was allowed to cool to 20-25°C and was stirred for 24 hours. The mixture was cooled to 0°C and then filtered. The residue was washed with acetonitrile (2x 10ml) and then dried *in vacuo* at 45°C for 2 hours to leave the title compound as a colourless solid (3.6g 69% yield, 95% pure by NMR).
¹H-NMR (CD₃OD) δ: 2.62-2.57 (1H, m), 2.53 (1H, dd, *J*14.8, 7.2Hz), 2.23 (1H, dd, *J* 14.8, 7.2Hz), 1.76-1.18 (15H, m), 1.44 (9H, s), 0.93-0.82 (2H, m)

### Preparation 2: Sodium 1-hydroxy-3-(2-methyl-1,1'-biphenyl-4-yl)-1-propanesulfonate

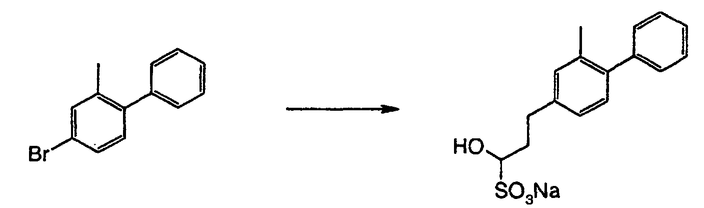

A stirred solution of 4-bromo-2-methyl-1,1'-biphenyl (20g, 81mmol), allyl alcohol (14ml, 0.20mol), tetrabutylammonium chloride (22g, 81mmol), sodium bicarbonate (17g, 0.20mol), palladium(II)acetate (0.91g, 4.0mmol) and tri-*o*-tolylphosphine (2.5g, 8.1mmol) in acetonitrile (200ml) was heated to reflux for 1 hour, under nitrogen, and then cooled. Ethyl acetate (200ml) was added and the mixture was washed with water (2x200ml), aqueous citric acid solution (10%, 100ml) and brine (100ml). Magnesium sulphate (20g) and charcoal (2g) were added and the mixture was filtered and concentrated *in vacuo* to an oil. The oil was taken up in methanol (100ml) and a solution of sodium metabisulfite (11.2g) in water (20ml) was added dropwise, over 10 min. The resulting mixture was stirred at 20-25°C for 16 hours and then filtered. The residue was washed with ethyl acetate (3x20ml) and dried *in vacuo* to leave the title compound as a solid (15.9g, 42% yield, 95% pure by NMR).
¹H-NMR (DMSO) δ : 7.49-7.30 (5H, m), 7.11-7.04 (3H, m), 5.26 (1H, br d), 3.84-3.78 (1H, m), 2.81-2.70 (1H, m), 2.20 (3H, s), 2.12-1.99 (1H, m), 1.85-1.74 (1H, m)

### Example 10: (E)-2-[2-(tert-butoxy)-2-oxoethyl]-5-(2-methyl-1.1'-biphenyl-4-yl)-2-pentenoic acid cyclohexylamine salt

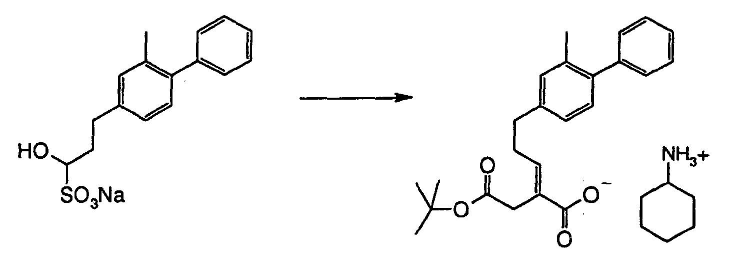

A solution of potassium *tert*-butoxide (6.6Kg, 59 mol) in THF (22 litres) was added over 1 hour to a stirred solution of sodium 1-hydroxy-3-(2-methyl-1,1'-biphenyl-4-yl)-1-propanesulfonate (4.55Kg, 13.8 mol) and 3-(diethoxyphosphoryl)succinic acid 1-*tert*-butyl ester (4.94Kg, 15.9 mol) in THF (5.2 litres) and *tert*-butanol (18.3 litres), from -5 to 0°C, under nitrogen. The mixture was stirred from -5 to 0°C for 4 hours and then a solution of citric acid (12.0Kg, 62 mol) in demineralised water (28 litres) was added in one portion. The pH was adjusted to pH4-5 by the addition of aqueous sodium hydroxide solution (40%) and the organic phase was separated. The organic phase was concentrated *in vacuo* to a volume of approximately 25 litres and then recombined with the aqueous phase. Ethyl acetate (28 litres) was added and the organic phase was separated and then washed with a solution of sodium bicarbonate (3.18Kg) in demineralised water (45 litres). Demineralised water (15 litres) was added and the pH was adjusted to pH 4-5 by the addition of a solution of citric acid (2.27Kg) in demineralised water (23 litres). The organic phase was separated, washed with demineralised water (14 litres) and then azeotropically dried by distillation at atmospheric pressure at a constant volume of 56 litres. The reaction was cooled to 35 to 40°C and cyclohexylamine (1.10Kg, 11.1 mol) was added in one portion. The mixture was cooled to 20-25°C and was stirred for 18 hours. The mixture was then cooled to 0°C, and was stirred for 2 hours and then filtered. The residue was washed with ethyl acetate (5 litres), then dried *in vacuo* at 40-45°C to leave the title compound as a colourless solid (4.1 Kg, 61% yield, 89% pure by HPLC).
¹H-NMR (CDCl₃) δ: 7.42-7.30 (5H, m), 7.16 (1H, d, *J* 7.6Hz), 7.15-7.05 (2H, m), 6.83 (1H, t, J 7.2Hz), 3.29 (2H, s), 2.50-2.43 (2H, m), 2.26 3H, s), 2.03-1.98 (2H, m), 1.78-1.71 (2H, m), 1.61-1.57 (1H, m), 1.44 (9H, s), 1.30-1.10 (5H, m)

### Example 11: (E)-2-[2-(tert-butoxy)-2-oxoethyl]-5-(2-methyl-1,1'-biphenyl-4-yl)-2-pentenoic acid adamantanamine salt

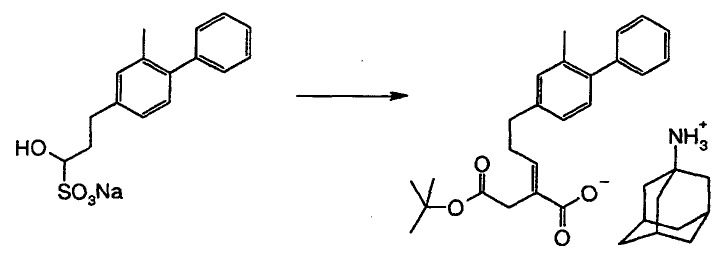

A solution of potassium phosphate tribasic (160g, 0.762 moles) in demineralised water (500ml) was added over 15 minutes to a stirred slurry of sodium 1-hydroxy-3-(2-methyl-1,1'-biphenyl-4-yl)-1-propanesulfonate (125g, 0.381 moles) in toluene (1500ml) and demineralised water (1000ml). The mixture was stirred at 20-25°C for 16 hours. The organic phase was separated and the aqueous phase was extracted with toluene (100ml). The combined organic extracts were washed with demineralised water (1000ml), and the solution was azeotropically dried by distillation of toluene at 40°C under reduced pressure. The volume of solution was reduced to 250ml, and allowed to cool to 20-25°C. Meanwhile, 3-(diethoxyphosphoryl)succinic acid 1-*tert*-butyl ester (112g, 0.360moles) was added portionwise over 10 minutes to a solution of sodium *tert*-butoxide (114g, 1.175moles) in toluene (1120ml) at -10°C under nitrogen. The mixture was stirred for 30 minutes at -10°C. The toluene solution prepared previously was added over 45 minutes between -10°C and 0°C. The mixture was stirred at -10°C for 1 hour and aqueous citric acid solution (10%w/v, 1000ml) was added. The biphasic mixture was stirred at 20-25°C for 16 hours. The organic phase was separated and washed with demineralised water (1000ml). The organic phase was azeotropically dried by distillation at 40°C under reduced pressure at a constant volume of 1330ml. The solution was maintained at 40-45°C, and a solution of adamantanamine (53g, 0.350moles) in toluene (665ml) was added in one portion. The mixture was cooled to 20-25°C, and was stirred for 16 hours. The precipitate was collected by filtration, washed with toluene (150ml) and dried *in vacuo* at 50°C to leave the title compound as a colourless solid (153g, 76% yield).
¹H-NMR (CDCl₃) δ: 7.42-7.05 (8H, m), 6.91 (1H, t, *J* 7.2Hz), 3.29 (2H, s), 2.79-2.71 (2H, m), 2.52-2.43 (2H, m), 2.25 (3H, s),2.08 (3H, s), 1.81 (6H s), 1.65 (6H s), 1.42 (9H, s),

### Example 12: (E)-2-[2-(tert-butoxy)-2-oxoethyl]-5-(2-methyl-1,1'-biphenyl-4-yl)-2-pentenoic acid sodium salt

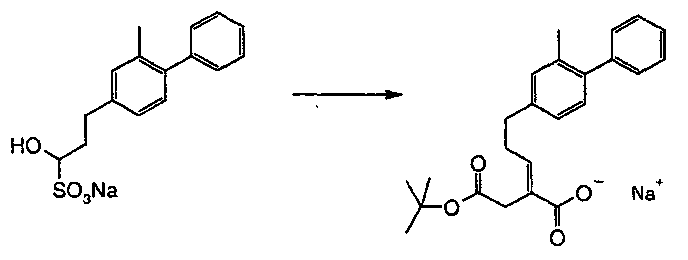

A solution of potassium *tert-*butoxide (7.25g, 64.6mmol) in THF (24ml) was added over 1 hour to a stirred solution of sodium 1-hydroxy-3-(2-methyl-1,1'-biphenyl-4-yl)-1-propanesulfonate (5.0g, 15.2mmol) and 3-(diethoxyphosphoryl)succinic acid 1-*tert-*butyl ester (5.5g, 17.7mmol) in THF (6ml) and *tert*-butanol (30ml), between -5 and 0°C, under nitrogen. The mixture was stirred between -5 and 0°C for 4 hours and then a solution of citric acid (13.2g) in demineralised water (132ml) was added in one portion.

The pH was adjusted to pH4-5 by the addition of aqueous sodium hydroxide solution (40%) and the organic phase was separated. The organic phase was concentrated *in vacuo* and then recombined with the aqueous phase. Ethyl acetate (55ml) was added and the organic phase was separated and then washed with a solution of sodium bicarbonate (3.5g) in demineralised water (50ml). The organic phase was separated and washed with citric acid (5.0g) in demineralised water (50ml), demineralised water (50ml) and then concentrated *in vacuo* to an orange oil. The oil was taken up in acetonitrile (30ml) and a solution of sodium bicarbonate (0.65g, 4.1mmol) in demineralised water (5ml) was added. The solution was azeotropically dried by distillation at a constant volume of acetonitrile and the mixture was granulated at 20-25°C overnight. The mixture was filtered and the residue dried *in vacuo* at 45°C to give the title compound as a white solid (2.6g, 43% yield, 95% pure by NMR).
¹H-NMR (CDCl₃) δ: 7.38-7.21 (5H, m), 7.10 (1H, d, *J* 7.6Hz), 7.05 (1H, s), 7.02(1H, d *J* 7.2Hz), 6.89 (1H, t, *J* 7.2Hz), 3.30 (2H, s), 2.70-2.65 (2H, m), 2.47-2.41 (2H, m), 2.19 (3H, s), 1.40 (9H, s).

### Example 13: (R)-2-[2-(tert-butoxy)-2-oxoethyl]-5-(2-methyl-1,1'-biphenyl-4-yl)-pentanoic acid

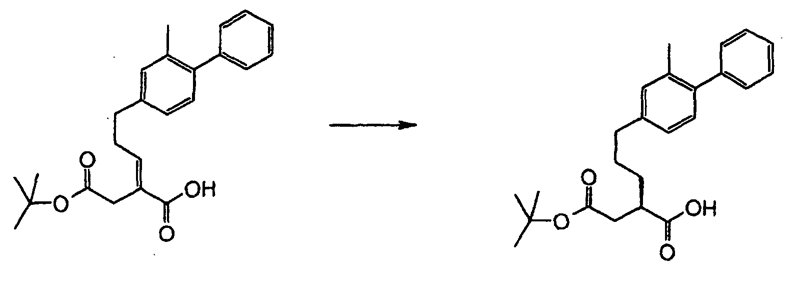

A solution of (*E*)-2-[2-(*tert*-butoxy)-2-oxoethyl]-5-(2-methyl-1,1'-biphenyl-4-yl)-2-pentenoic acid (3.8g, 10 mmol) and 1,1'-bis[(2*S*,4*S*)-2,4-diethylphosphetano]ferrocene-(1,5-cyclooctadiene)-rhodium (I) tetrafluoroborate (7.8mg, 10µmol) in methanol (10ml) was stirred at 20-25°C for 24 hours, under hydrogen (60p.s.i.). The mixture was then concentrated *in vacuo* to leave the title compound as a yellow oil (3.8g, 98% conversion, enantiomeric excess=95%, 95% pure by NMR).
¹H-NMR (CDCl₃) δ : 7.42-7.30 (5H, m), 7.18-7.03 (3H, m), 2.94-2.82 (1H, m), 2.70-2.62 (3H, m), 2.41 (1H, br dd), 2.23 (3H, s), 1.80-1.59 (4H, m), 1.43 (9H, s)

### Example 14: (R)-2-[2-(tert-butoxy)-2-oxoethyl]-5-(2-methyl-1,1'-biphenyl-4-yl)-pentanoic acid cyclohexylamine salt

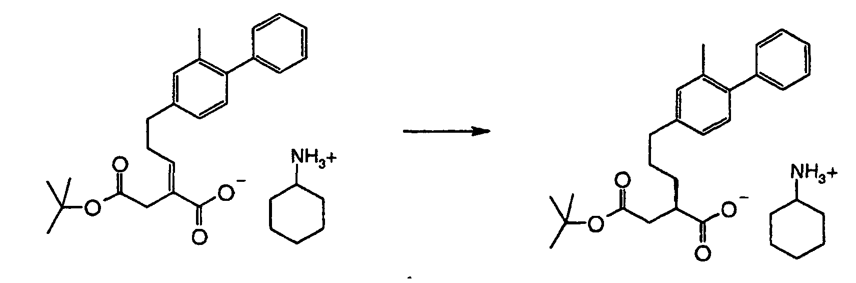

A stirred solution of (*E*)-2-[2-(*tert*-butoxy)-2-oxoethyl]-5-(2-methyl-1,1'-biphenyl-4-yl)-2-pentenoic acid cyclohexylamine salt (1.1Kg, 2.3 mol) and [(*S*)-2,2'-bis(diphenylphosphino-1,1'-binaphthyl]chloro(*p*-cymene)ruthenium chloride (2.2g, 2.4 mmol) in methanol (8.2 litres) and water (2.8 litres) was heated to 60°C, under hydrogen (60p.s.i.), for 40 hours and then allowed to cool to 20-25°C (enantiomeric excess= 88%). The mixture was concentrated *in vacuo* to a volume of 3 litres and then ethyl acetate (5 litres) was added. The mixture was distilled at constant volume of ethyl acetate until water droplets appeared in the distillate. The mixture was then cooled to 20-25°C and then demineralised water (2.9 litres) and citric acid (485g, 2.5 mol) were added. The organic phase was separated and washed with demineralised water (1.1 litre) and then dried azeotropically by distillation at a constant volume of 8.25 litres. Methylethyl ketone (8.25 litres) was added and the mixture was warmed to 40°C. Cyclohexylamine (228g, 2.28mol) was added in one portion and the mixture was allowed to cool to 20-25°C, then was stirred for 16 hours. The mixture was filtered and the residue was washed with a mixture of ethyl acetate (55ml) and methylethyl ketone (55ml) and then dried *in vacuo* at 45°C to leave the title compound as a colourless solid (0.71 Kg, 65% yield, enantiomeric excess=98.6%, 99% pure by HPLC).
¹H-NMR (CDCl₃) δ : 7.42-7.25 (5H, m), 7.11 (1H, d, *J* 7.6Hz), 7.08-7.00 (2H, m), 2.90-2.82 (1H, m), 2.67-2.58 (4H, m), 2.30 (1H, br dd), 2.23 (3H, s), 2.00-1.86 (2H, m), 1.80-1.50 (7H, m), 1.41 (9H, s), 1.38-1.09 (5H, m)

### Example 15: (R)-2-[2-(tert-butoxy)-2-oxoethyl]-5-(2-methyl-1,1'-biphenyl-4-yl)-pentanoic acid (S)-alpha-methylbenzylamine salt

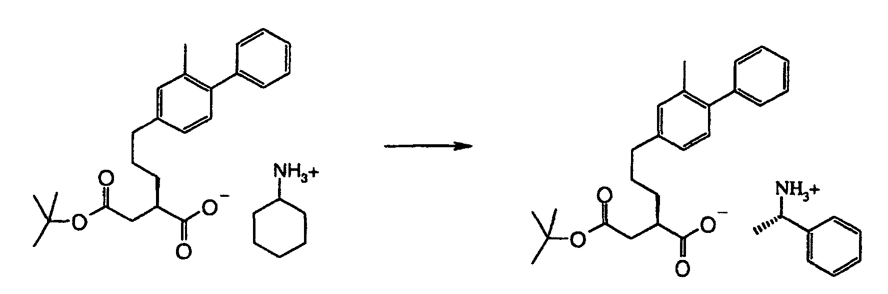

(*R*)-2-[2-(*tert*-butoxy)-2-oxoethyl]-5-(2-methyl-1,1'-biphenyl-4-yl)-pentanoic acid can be enantiomerically upgraded as the (*S*)-*alpha*-methylbenzylamine salt. Thus, (*R*)-2-[2-(*tert*-butoxy)-2-oxoethyl]-5-(2-methyl-1,1'-biphenyl-4-yl)-pentanoic acid cyclohexylamine salt (50g, 0.10 mol, enantiomeric excess= 72%) was partitioned between ethyl acetate (750ml) and aqueous citric acid solution (10%, 750ml). The organic phase was separated, washed with water (500ml), then azeotropically dried by distillation at constant volume. (*S*)-*alpha*-methylbenzylamine (13.2ml, 0.10 mol) was added dropwise over 5 min at 40°C, the mixture was allowed to cool to 20-25°C, and was then stirred for 24 hours. The mixture was filtered and the residue was washed with ethyl acetate (100ml) and then dried *in vacuo*) at 45°C for 2 hours to leave the title compound as a colourless solid (41.0g, 91% yield, enantiomeric excess= 96.4%, 95% pure by NMR).
¹H-NMR (CDCl₃) δ : 7.40-7.22 (10H, m), 7.12 (1H, d, *J* 7.6Hz), 7.06-7.02 (2H, m), 4.22-4.18 (1H, m), 2.80-2.76 (1H, m), 2.63-2.59 (3H, m), 2.34 (1H, dd, *J* 16.4, 5.6Hz), 2.24 (3H, s), 1.77-1.70 (3H, m), 1.61-1.56 (1H, m), 1.47 (3H, d, *J* 6.8Hz), 1.40 (9H, s)

## Claims

1. A process for the preparation of a compound of the formula (IV), (V) or (VI): 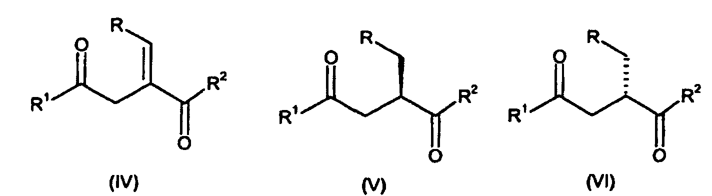 wherein R is aryl, C₃₋₈ cycloalkyl, C₁₋₁₀ alkyl, (aryl)C₁₋₁₀ alkylene, (C₃₋₈ cycloalkyl)C₁₋₁₀ alkylene, heterocyclyl, (heterocyclyl)C₁₋₁₀ alkylene, (aryl)C₃₋₈ cycloalkylene, (C₃₋₈ cycloalkyl)arylene or (C₁₋₁₀ alkylaryl)C₁₋₁₀ alkylene,
wherein "aryl" is a mono- or bicyclic partially or fully unsaturated carbocyclic ring system containing from 4 to 10 atoms, or a partially or fully unsaturated mono- or bicyclic heterocyclic moiety having up to 10 atoms in the ring system and with up to 4 hetero-atoms in the said ring system each independently selected from N, O and S,
said carbocyclic ring system and heterocyclic moiety being optionally substituted by one or more substituents each independently selected from halogen, NO₂, NH₂, CO₂R⁹, phenyl, C₁₋₆ alkyl(optionally substituted by one or more halogen), and C₁₋₆ alkoxy(optionally substituted by one or more halogen), and
"heterocyclyl" is a 3- to 8-membered mono or bicyclic saturated heterocyclic group having from 1 to 4 ring hetero-atoms each independently selected from N, O and S, optionally substituted by one or more substituents each independently selected from halogen, NO₂, NH₂, CO₂R⁹, phenyl, C₁₋₆ alkyl(optionally substituted by one or more halogen), and C₁₋₆ alkoxy(optionally substituted by one or more halogen);
R¹ is C₁₋₆ alkoxy;
R² is OH or O⁻M⁺;
R⁹ is H or C₁₋₆ alkyl;
and M⁺ is the cation of a metal or is a protonated amine moiety;
which comprises:
formation of a compound of formula (IV) as defined above, by reaction of an aldehyde of formula RCHO, or a protected derivative thereof or adduct thereof, wherein R is as defined above,
with a phosphorus compound of formula (IX): 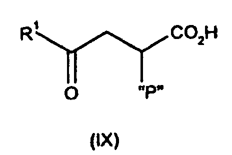
or a metal carboxylate salt thereof, wherein R¹ is as defined above, and
"P" is a phosphonate moiety of formula -P(O)(OR³)(OR⁴), wherein R³ and R⁴ are either each independently selected from H, C₁₋₆ alkyl, benzyl and phenyl (optionally substituted by one or more C₁₋₆ alkyl), or R³ and R⁴ taken together are C₂₋₅ alkylene, or "P" is a phosphorane moiety of formula -(PR⁵R⁶R⁷)⁺X⁻ wherein R⁵, R⁶ and R⁷ are each independently selected from C₁₋₆ alkyl and phenyl,
and X is bromine, chlorine or iodine,
in the presence of a sodium, lithium or potassium C₁-C₆ alkoxide base,
in an inert solvent, and
at a temperature of from -80°C to 20°C,
said process being optionally followed by conversion of the product of formula (IV) where R² is O⁻M⁺ wherein M⁺ is a metal to a compound of formula (IV) where R² is OH by treatment with a proton source, which may optionally be converted to a compound of formula (IV) wherein R² is O⁻M⁺(where M⁺ is a protonated amine moiety as previously defined), by treatment with a corresponding amine, optionally followed by, if a compound of formula (V) or (VI) is desired, asymmetric reduction of a compound of formula (IV) to provide a compound of formula (V) or (VI) with the proviso that R³ and R⁴ are not simultaneously ethyl when R is -(CH₂)₂-C₆H₅ and R¹ is t-butoxy.

2. A process according to claim 1 wherein the olefination is carried out in a reaction time of less than 24 hours.

3. A process according to claim 1 or 2, where R is (aryl)C₁₋₁₀ alkylene or (C₃₋₈ cycloalkyl)C₁₋₁₀ alkylene.

4. A process according to claim 3, where R is phenylethyl, cyclohexylethyl or (2-methyl-1,1'-biphenyl-4-yl)ethyl.

5. A process according to any preceding claim, where R¹ is t-butoxy.

6. A process according to any preceding claim, where R² is OH, O⁻Li⁺, O⁻Na⁺ , O⁻K⁺, O⁻cyclohexylammonium⁺, O⁻adamantanammonium⁺, O⁻ triethanolammonium⁺ or O⁻(*S*)-α-methylbenzylammonium⁺.

7. A process according to any preceding claim, where the olefination reaction is carried out using the aldehyde RCHO or the sodium bisulphite adduct thereof RCH(OH)SO₃⁻Na⁺.

8. A process according to any preceding claim, where "P" is P(O)(OC₂H₅)₂, P(O)(OCH₂CH₂O) or a triphenylphosphinium halide moiety.

9. A process according to claim 8, where "P" is P(O)(OC₂H₅)₂.

10. A process according to claim 1, where the base is potassium t-butoxide, sodium t-butoxide or sodium methoxide.

11. A process according to any preceding claim, where the olefination reaction solvent is anhydrous tetrahydrofuran, anhydrous toluene or anhydrous R¹H, or a mixture thereof.

12. A process according to claim 11, where the reaction solvent is selected from tetrahydrofuran and toluene when the aldehyde RCHO is used as a substrate, and selected from tetrahydrofuran, t-butanol and toluene when the bisulphite adduct is used as substrate.

13. A process according to any preceding claim, where the olefination reaction is carried out at a temperature of from -20°C to 10°C.

14. A process according to claim 13, where the reaction is carried out at a temperature of from -10°C to 10°C.

15. A process according to claim 14, where the reaction is carried out at a temperature of from 0°C to 5°C.

16. A process according to claim 1, where in the olefination reaction, the aldehyde RCHO is used as substrate, and the reaction is carried out by adding the phosphorus compound to the base and solvent mixture, followed by addition of the aldehyde.

17. A process according to claim 1, where in the olefination reaction, the aldehyde RCHO is used as substrate, and the reaction is carried out by combining the phosphorus compound and the aldehyde, followed by addition of the base.

18. A process according to claim 1, where in the olefination reaction, the aldehyde RCHO is used as substrate, and the reaction is carried out by adding the base to the phosphorus compound followed by addition of the aldehyde.

19. A process according to claim 1, where in the olefination reaction, the bisulphite is used as the substrate, and the reaction is carried out by adding the base to a mixture of the bisulphite adduct and the phosphorus compound.

20. A process according to claim 1, where in the olefination reaction, the bisulphite is used as the substrate, and the reaction is carried out by adding the bisulphite to a mixture of the phosphorus compound and the base.

21. A process according to claim 1 for the preparation of a compound of formula (V) or (VI) which comprises the asymmetric hydrogenation of compounds of the formula (IV) as defined in claim 1.

22. A process according to claim 1 or claim 21, where the catalyst used for reduction of compounds of formula (IV) where R² is O⁻M⁺, is ruthenium based.

23. A process according to claim 1 or claim 22, where the catalyst used is a ruthenium complex of BINAP or a derivative thereof.

24. A process according to claim 1 or claim 23, where the catalyst used is [(*S*)-2,2'-bis(diphenylphosphino-1,1'-binaphthyl]chloro(*p*-cymene)ruthenium chloride.

25. A process according to claim 1 or claim 21, where the catalyst used for reduction of compounds of formula (IV) where R² is OH, is rhodium-based.

26. A process according to claim 1 or claim 25, where the catalyst used is Rh-DUPHOS (1,2-bis[(2S,5S)-2,5-diethylphospholano]benzene-(1,5-cyclooctadien)-rhodium (I) tetrafluoroborate), or Rh-Ferrotane (1,1'-bis[(2S,4S)-2,4-diethylphosphetano]ferrocene-(1,5-cyclooctadiene)-rhodium (I) tetrafluoroborate).

27. A process according to claim 1 or claim 21, where the hydrogenation of the acid (IV, R² is OH) is carried out in the presence of a base.

28. A process according to claim 27, where the base is sodium bicarbonate, cyclohexylamine, isopropylamine, t-butylamine, adamantanamine or (S)-α-methylbenzylamine.

29. A process according to claim 1 or claim 21, where the hydrogenation can be carried out on a preformed salt (IV, R² is O⁻M⁺).

30. A process according to claim 1 or claim 21, where the hydrogenation is carried out in an inert solvent, under a positive pressure of hydrogen.

31. A process according to claim 30, wherein the solvent is an aqueous C₁₋₃ alcohol or a C₁₋₃ alcohol.

32. A process according to claim 1 or claim 21, where the reaction is carried out with a ruthenium-based catalyst and the reaction temperature is approximately 60°C.

33. A process according to claim 1 or claim 21, where the reaction is carried out with a rhodium-based catalyst and the reaction temperature is approximately 20°C.

## Patentansprüche

1. Verfahren zur Herstellung einer Verbindung der Formel (IV), (V) oder (VI): 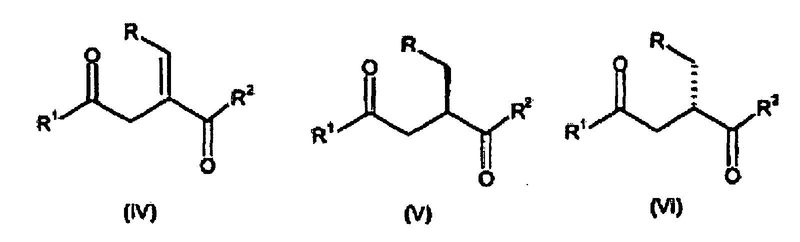 wobei R Aryl, C₃₋₈-Cycloalkyl, C₁₋₁₀-Alkyl, (Aryl)C₁₋₁₀-Alkylen, (C₃₋₈-Cycloalkyl)C₁₋₁₀-Alkylen, Heterocyclyl, (Heterocyclyl)C₁₋₁₀-Alkylen, (Aryl)C₃₋₈-Cycloalkylen, (C₃₋₈-Cycloalkyl)arylen oder (C₁₋₁₀-Alkylaryl)C₁₋₁₀-Alkylen ist,
wobei "Aryl" ein von 4 bis 10 Atome enthaltendes mono- oder bizyklisches teilweise oder vollständig ungesättigtes carbozyklisches Ringsystem oder eine teilweise oder vollständig ungesättigte mono- oder bizyklische heterozyklische Einheit mit bis zu 10 Atomen im Ringsystem und mit bis zu 4 je unabhängig aus N, O und S ausgewählten Heteroatomen im besagten Ringsystem ist,
wobei besagte(s) carbozyklisches Ringsystem und heterozyklische Einheit optional substituiert sind mit einem oder mehreren je unabhängig aus Halogen, NO₂, NH₂, CO₂R⁹, Phenyl, C₁₋₆-Alkyl (optional mit einem oder mehreren Halogenen substituiert) und C₁₋₆-Alkoxy (optional mit einem oder mehreren Halogenen substituiert) ausgewählten Substituenten, und
wobei "Heterocyclyl" eine 3- bis 8-gliedrige mono- oder bizyklische gesättigte heterozyklische Gruppe mit 1 bis 4 je unabhängig aus N, O und S ausgewählten Ringheteroatomen ist, die optional substituiert ist mit einem oder mehreren je unabhängig aus Halogen, NO₂, NH₂, CO₂R⁹, Phenyl, C₁₋₆-Alkyl (optional mit einem oder mehreren Halogenen substituiert) und C₁₋₆-Alkoxy (optional mit einem oder mehreren Halogenen substituiert) ausgewählten Substituenten;
R¹ C₁₋₆-Alkoxy ist;
R² OH oder O⁻M⁺ ist;
R⁹ H oder C₁₋₆-Alkyl ist;
und M⁺ das Kation eines Metalles oder eine protonierte Amin-Einheit ist;
welches umfasst:
Bildung einer Verbindung der Formel (IV) wie oben definiert durch Umsetzen eines Aldehydes der Formel RCHO, oder eines geschützten Derivates davon oder Adduktes davon, wobei R wie oben definiert ist,
mit einer Phosphorverbindung der Formel (IX): 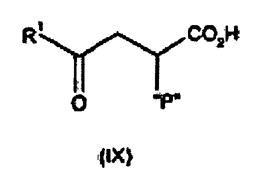
oder einem Metallcarboxylat-Salz davon,
wobei R¹ wie oben definiert ist und
"P" eine Phosphonat-Einheit der Formel -P(O)(OR³)(OR⁴) ist, wobei R³ und R⁴ entweder je unabhängig aus H, C₁₋₆-Alkyl, Benzyl und Phenyl (optional substituiert mit einem oder mehreren C₁₋₆-Alkyl) ausgewählt sind, oder R³ und R⁴ zusammengenommen C₂₋₅-Alkylen sind,
oder "P" eine Phosphoran-Einheit der Formel -(PR⁵R⁶R⁷)⁺X⁻ ist, wobei R⁵, R⁶ und R⁷ je unabhängig aus C₁₋₆-Alkyl und Phenyl ausgewählt sind,
und X Brom, Chlor oder Iod ist,
in Gegenwart einer Natrium-, Lithium- oder Kalium-C₁-C₆-Alkoxidbase,
in einem inerten Lösungsmittel, und
bei einer Temperatur von -80°C bis 20°C,
wobei besagtes Verfahren optional gefolgt wird von der Umwandlung des Produktes der Formel (IV) wobei R² O⁻M⁺ ist,
wobei M⁺ ein Metall ist, in eine Verbindung der Formel (IV)
wobei R² OH ist, durch Behandlung mit einer Protonenquelle, welches optional in eine Verbindung der Formel (IV) umgewandelt werden kann, wobei R² O⁻M⁺ ist (wobei M⁺ eine protonierte Amin-Einheit wie vorgängig definiert ist) durch Behandlung mit einem entsprechenden Amin;
optional gefolgt, falls eine Verbindung der Formel (V) oder (VI) gewünscht ist, von einer asymmetrischen Reduktion einer Verbindung der Formel (IV), unter Bildung einer Verbindung der Formel (V) oder (VI), mit der Massgabe, dass R³ und R⁴ nicht gleichzeitig Ethyl sind, wenn gilt: R ist -(CH₂)₂-C₆H₅ und R¹ ist t-Butoxy.

2. Verfahren nach Anspruch 1, wobei die Olefinierung in einer Reaktionszeit von weniger als 24 Stunden durchgeführt wird.

3. Verfahren nach Anspruch 1 oder 2, wobei R (Aryl)C₁₋₁₀alkylen oder (C₃₋₈-Cycloalkyl)C₁₋₁₀-alkylen ist.

4. Verfahren nach Anspruch 3, wobei R Phenylethyl, Cyclohexylethyl oder (2-Methyl-1,1'-biphenyl-4-yl)ethyl ist.

5. Verfahren nach irgendeinem der vorangehenden Ansprüche, wobei R¹ t-Butoxy ist.

6. Verfahren nach irgendeinem der vorangehenden Ansprüche, wobei R² OH, O⁻Li⁺, O⁻Na⁺, O⁻K⁺, O-Cyclohexylammonium⁺, O-Adamantanammonium⁺, O⁻Triethanolammonium⁺ oder O⁻(S)-α-Methylbenzylammonium⁺ ist.

7. Verfahren nach irgendeinem der vorangehenden Ansprüche, wobei die Olefinierungsreaktion unter Verwendung des Aldehydes RCHO oder des Natriumbisulfit-Adduktes davon RCH(OH)SO₃⁻Na⁺ durchgeführt wird.

8. Verfahren nach irgendeinem der vorangehenden Ansprüche, wobei "P" P(O)(OC₂H₅)₂, P(O)(OCH₂CH₂O) oder eine Triphenylphosphiniumhalogenid-Einheit ist.

9. Verfahren nach Anspruch 8, wobei "P" P(O)(OC₂H₅)₂ ist.

10. Verfahren nach Anspruch 1, wobei die Base Kalium-tbutoxid, Natrium-t-butoxid oder Natriummethoxid ist.

11. Verfahren nach irgendeinem der vorangehenden Ansprüche, wobei das Lösungsmittel der Olefinierungsreaktion wasserfreies Tetrahydrofuran, wasserfreies Toluol oder wasserfreies R¹H, oder ein Gemisch davon ist.

12. Verfahren nach Anspruch 11, wobei das Lösungsmittel der Reaktion aus Tetrahydrofuran und Toluol ausgewählt ist, wenn das Aldehyd RCHO als Substrat verwendet wird, und aus Tetrahydrofuran, t-Butanol und Toluol ausgewählt ist, wenn das Bisulfit-Addukt als Substrat verwendet wird.

13. Verfahren nach irgendeinem der vorangehenden Ansprüche, wobei die Olefinierungsreaktion bei einer Temperatur von -20°C bis 10°C durchgeführt wird.

14. Verfahren nach Anspruch 13, wobei die Reaktion bei einer Temperatur von -10°C bis 10°C durchgeführt wird.

15. Verfahren nach Anspruch 14, wobei die Reaktion bei einer Temperatur von 0°C bis 5°C durchgeführt wird.

16. Verfahren nach Anspruch 1, wobei in der Olefinierungsreaktion das Aldehyd RCHO als Substrat verwendet wird und die Reaktion durch Zugabe der Phosphorverbindung zur Base und zum Lösungsmittelgemisch, gefolgt von der Zugabe des Aldehydes, durchgeführt wird.

17. Verfahren nach Anspruch 1, wobei in der Olefinierungsreaktion das Aldehyd RCHO als Substrat verwendet wird und die Reaktion durch Kombinieren der Phosphorverbindung und des Aldehydes, gefolgt von der Zugabe der Base, durchgeführt wird.

18. Verfahren nach Anspruch 1, wobei in der Olefinierungreaktion das Aldehyd RCHO als Substrat verwendet wird und die Reaktion durch Zugabe der Base zur Phosphorverbindung, gefolgt von der Zugabe des Aldehydes, durchgeführt wird.

19. Verfahren nach Anspruch 1, wobei in der Olefinierungsreaktion das Bisulfit als das Substrat verwendet wird und die Reaktion durch Zugabe der Base zu einem Gemisch des Bisulfit-Adduktes und der Phosphorverbindung durchgeführt wird.

20. Verfahren nach Anspruch 1, wobei in der Olefinierungsreaktion das Bisulfit als das Substrat verwendet wird und die Reaktion durch Zugabe des Bisulfits zu einem Gemisch der Phosphorverbindung und der Base durchgeführt wird.

21. Verfahren nach Anspruch 1 zur Herstellung einer Verbindung der Formel (V) oder (VI), welches die asymmetrische Hydrierung von Verbindungen der Formel (IV) wie in Anspruch 1 definiert umfasst.

22. Verfahren nach Anspruch 1 oder Anspruch 21, wobei der für die Reduktion der Verbindungen der Formel (IV), wobei R² O⁻M⁺ ist, verwendete Katalysator auf Ruthenium beruht.

23. Verfahren nach Anspruch 1 oder Anspruch 22, wobei der verwendete Katalysator ein Rutheniumkomplex von BINAP oder ein Derivat davon ist.

24. Verfahren nach Anspruch 1 oder Anspruch 23, wobei der verwendete Katalysator [(S)-2,2'-Bis(diphenylphosphino-1,1'binaphthyl]chloro(p-cymen)Rutheniumchlorid ist.

25. Verfahren nach Anspruch 1 oder Anspruch 21, wobei der für die Reduktion der Verbindungen der Formel (IV), wobei R² OH ist, verwendete Katalysator auf Rhodium beruht.

26. Verfahren nach Anspruch 1 oder Anspruch 25, wobei der verwendete Katalysator Rh-DUPHOS (1,2-bis[(2S,5S)-2,5-Diethylphospholano]benzol-(1,5-cyclooctadien)-rhodium (I) tetrafluoroborat) oder Rh-Ferrotan (1,1'-bis[(2S,4S)-2,4-Diethylphosphetano]ferrocen-(1,5-cyclooctadien)-rhodium (I) tetrafluoroborat) ist.

27. Verfahren nach Anspruch 1 oder Anspruch 21, wobei die Hydrierung der Säure (IV, R² ist OH) in Gegenwart einer Base durchgeführt wird.

28. Verfahren nach Anspruch 27, wobei die Base Natriumbicarbonat, Cyclohexylamin, Isopropylamin, t-Butylamin, Adamantanamin oder (S)-α-Methylbenzylamin ist.

29. Verfahren nach Anspruch 1 oder Anspruch 21, wobei die Hydrierung auf einem vorgängig gebildeten Salz (IV, R² ist O⁻ M⁺) durchgeführt werden kann.

30. Verfahren nach Anspruch 1 oder Anspruch 21, wobei die Hydrierung in einem inerten Lösungsmittel unter einem positiven Wasserstoffdruck durchgeführt wird.

31. Verfahren nach Anspruch 30, wobei das Lösungsmittel ein wässriger C₁₋₃-Alkohol oder ein C₁₋₃-Alkohol ist.

32. Verfahren nach Anspruch 1 oder Anspruch 21, wobei die Reaktion mit einem auf Ruthenium beruhenden Katalysator durchgeführt wird und die Reaktionstemperatur ungefähr 60°C beträgt.

33. Verfahren nach Anspruch 1 oder Anspruch 21, wobei die Reaktion mit einem auf Rhodium beruhenden Katalysator durchgeführt wird und die Reaktionstemperatur ungefähr 20°C beträgt.

## Revendications

1. Procédé de préparation d'un composé de formule (IV), (V) ou (VI) : 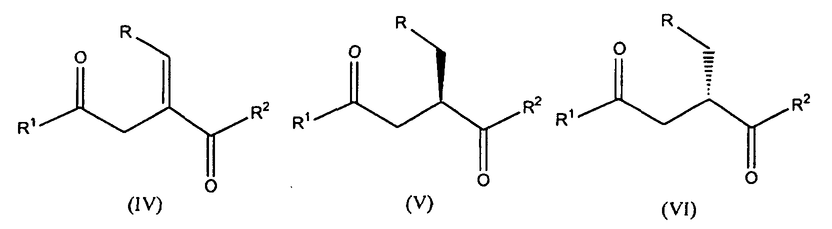 dans lesquelles R représente aryle, cycloalkyle en C₃₋₈, alkyle en C₁₋₁₀, (aryl)alkylène en C₁₋₁₀, (cycloalkyle en C₃₋₈) alkylène en C₁₋₁₀, hétérocyclyle, (hétérocyclyl)alkylène en C₁₋₁₀, (aryl)cycloalkylène en C₃₋₈, (cycloalkyle en C₃₋₈)arylène ou [(alkyle en C₁₋₁₀)aryl] alkylène en C₁₋₁₀, ou "aryle" représente un système de noyaux carbocycliques mono- ou bicycliques partiellement ou totalement insaturés, contenant de 4 à 10 atomes de carbone, ou un motif hétérocyclique mono- ou bicyclique partiellement ou totalement insaturé ayant jusqu'à 10 atomes de carbone dans le système de noyaux et ayant jusqu'à 4 hétéroatomes dans ledit système de noyaux, dont chacun est indépendamment choisi parmi N, O et S, ledit système de noyaux carbocycliques et ledit motif hétérocyclique étant facultativement substitués par un ou plusieurs substituants dont chacun est indépendamment choisi parmi halogène, NO₂, NH₂, CO₂R⁹, phényle, alkyle en C₁₋₆ (facultativement substitué par un ou plusieurs halogènes), et alkoxy en C₁₋₆ (facultativement substitué par un ou plusieurs halogènes), et
"hétérocyclyle" représente un groupe hétérocyclique saturé mono- ou bicyclique, ayant de 3 à 8 éléments, avec de 1 à 4 hétéroatomes de cycle, dont chacun est indépendamment sélectionné parmi N, O et S, facultativement substitué par un ou plusieurs substituants dont chacun est indépendamment sélectionné parmi halogéno, NO₂, NH₂, CO₂R⁹, phényle, alkyle en C₁₋₆ (facultativement substitué par un ou plusieurs halogènes), et alkoxy en C₁₋₆ (facultativement substitué par un ou plusieurs halogènes) ;
R¹ représente alkoxy en C₁₋₆ ;
R² représente OH ou O⁻ M⁺ ;
R⁹ représente H ou alkyle en C₁₋₆ ;
et M⁺ est le cation d'un métal ou est un motif amine protonée ;
qui comprend :
la formation d'un composé de formule (IV) telle que définie ci-dessus, par réaction d'un aldéhyde de formule RCHO, ou d'un dérivé protégé ou d'un produit d'addition d'un tel aldéhyde, où R est tel que défini ci-dessus,
avec un composé du phosphore de formule (IX) : 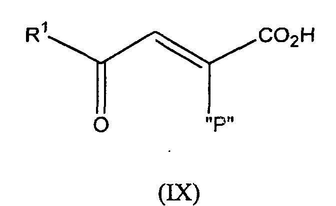 ou un carboxylate métallique d'un tel composé, où R¹ est tel que défini ci-dessus, et
"P" représente un motif phosphonate de formule -P(O)(OR³)(OR⁴), où soit R³ et R⁴ sont chacun indépendamment sélectionnés parmi H, alkyle en C₁₋₆, benzyle ou phényle (facultativement substitués par un ou plusieurs groupes alkyle en C₁₋₆), soit R³ et R⁴, pris ensemble, représentent alkylène en C₂₋₅,
ou "P" est un motif phosphorane de formule -(PR⁵R⁶R⁷) ⁺X⁻ où R⁵, R⁶ et R⁷ sont chacun indépendamment sélectionnés parmi alkyle en C₁₋₆ et phényle,
et X représente le brome, le chlore ou l'iode,
en présence d'un alcanolate en C₁-C₆ de sodium, de lithium ou de potassium comme base,
dans un solvant inerte, et
à une température comprise entre -80°C et 20°C,
ledit procédé étant facultativement suivi par la transformation du produit de formule (IV) ou R² représente O⁻M⁺, formule dans laquelle M⁺ est un métal, en un composé de formule (IV) dans laquelle R² représente OH, par traitement avec une source de protons, composé qui peut facultativement être transformé en un composé de formule (IV) dans laquelle R² représente O⁻M⁺ (où M⁺ est un motif amine protonée tel que précédemment défini), par traitement avec une amine correspondante,
facultativement suivi, si l'on désire un composé de formule (V) ou (VI), par une réduction asymétrique d'un composé de formule (IV) pour donner un composé de formule (V) ou (VI), étant entendu que R³ et R⁴ ne représentent pas simultanément éthyle lorsque que R représente -(CH₂)₂-C₆H₅ et R¹ représente t-butoxy.

2. Procédé selon la revendication 1, dans lequel l'oléfination est mise en oeuvre pendant un temps de réaction inférieur à 24 heures.

3. Procédé selon la revendication 1 ou 2, dans lequel R représente (aryl)alkylène en C₁₋₁₀ ou (cycloalkyle en C₃₋₈)alkylène en C₁₋₁₀.

4. Procédé selon la revendication 3, dans lequel R représente phénéthyle, cyclohexyléthyle ou (2-méthyl-1,1'-biphényl-4-yl)éthyle.

5. Procédé selon l'une quelconque des revendications précédentes, dans lequel R¹ représente t-butoxy.

6. Procédé selon l'une quelconque des revendications précédentes, dans lequel R² représente OH, O⁻Li⁺, O⁻Na⁺, O⁻ K⁺, O⁻ cyclohexylammonium⁺, O⁻ adamantanammonium⁺, O⁻ triéthanolammonium⁺ ou O⁻ (*S*)-α-méthylbenzylammonium⁺.

7. Procédé selon l'une quelconque des revendications précédentes, dans lequel la réaction d'oléfination est mise en oeuvre en utilisant l'aldéhyde RCHO ou le produit d'addition bisulfite de sodium d'un tel aldéhyde RCH (OH) SO₃⁻ Na⁺.

8. Procédé selon l'une quelconque des revendications précédentes, dans lequel "P" représente P (O) (OC₂H₅)₂, P(O)(OCH₂CH₂O) ou un motif halogénure de triphénylphosphinium

9. Procédé selon la revendication 8, dans lequel "P" représente P(O) (OC₂H₅)₂.

10. Procédé selon la revendication 1, dans lequel la base est le t-butanolate de potassium, le t-butanolate de sodium ou le méthanolate de sodium.

11. Procédé selon l'une quelconque des revendications précédentes, dans lequel le solvant de la réaction d'oléfination est le tétrahydrofurane anhydre, le toluène anhydre ou le R¹H anhydre, ou un mélange de ceux-ci.

12. Procédé selon la revendication 11, dans lequel le solvant de la réaction est sélectionné parmi le tétrahydrofurane et le toluène lorsque l'aldéhyde RCHO est utilisé comme substrat, et est sélectionné parmi le tétrahydrofurane, le t-butanol et le toluène lorsque le produit d'addition bisulfite est utilisé comme substrat.

13. Procédé selon l'une quelconque des revendications précédentes, dans lequel la réaction d'oléfination est mise en oeuvre à une température comprise entre -20°C et 10°C.

14. Procédé selon la revendication 13, dans lequel la réaction est mise en oeuvre à une température comprise entre -10°C et 10°C.

15. Procédé selon la revendication 14, dans lequel la réaction est mise en oeuvre à une température comprise entre 0°C et 5°C.

16. Procédé selon la revendication 1, dans lequel, dans la réaction d'oléfination, l'aldéhyde RCHO est utilisé comme substrat et la réaction est mise en oeuvre par adjonction du composé du phosphore au mélange de base et de solvant, suivie par l'addition de l'aldéhyde.

17. Procédé selon la revendication 1, dans lequel, dans la réaction d'oléfination, l'aldéhyde RCHO est utilisé comme substrat et la réaction est mise en oeuvre en combinant le composé du phosphore et l'aldéhyde, suivie par l'addition de la base.

18. Procédé selon la revendication 1, dans lequel, dans la réaction d'oléfination, l'aldéhyde RCHO est utilisé comme substrat et la réaction est mise en oeuvre en ajoutant la base au composé du phosphore, suivie par l'addition de l'aldéhyde.

19. Procédé selon la revendication 1, dans lequel, dans la réaction d'oléfination, le bisulfite est utilisé comme substrat et la réaction est mise en oeuvre par adjonction de la base à un mélange du produit d'addition bisulphite et du composé du phosphore.

20. Procédé selon la revendication 1, dans lequel, dans la réaction d'oléfination, le bisulfite est utilisé comme substrat et la réaction est mise en oeuvre par adjonction du bisulphite à un mélange du composé du phosphore et de la base.

21. Procédé selon la revendication 1 pour la préparation d'un composé de formule (V) ou (VI) qui comprend l'hydrogénation asymétrique de composés de formule (IV) tels que définis dans la revendication 1.

22. Procédé selon la revendication 1 ou 21, dans lequel le catalyseur utilisé pour la réduction des composés de formule (IV) lorsque R² représente O⁻M⁺, est à base de ruthénium.

23. Procédé selon la revendication 1 ou la revendication 22, dans lequel le catalyseur utilisé est un complexe de ruthénium du BINAP ou un dérivé de celui-ci.

24. Procédé selon la revendication 1 ou la revendication 23, dans lequel le catalyseur utilisé est le chlorure de [(S)-2,2'-bis(diphénylphosphino)-1,1'-binaphtyl]chloro(p-cymène)ruthénium.

25. Procédé selon la revendication 1 ou la revendication 21, dans lequel le catalyseur utilisé pour la réduction des composés de formule (IV) dans laquelle R² représente OH est à base de rhodium.

26. Procédé selon la revendication 1 ou la revendication 25, dans lequel le catalyseur utilisé est le Rh-DUPHOS (tétrafluoroborate de 1,2-bis[(2S,5S)-2,5-diéthylphospholano]benzène-(1,5-cyclooctadiène)-rhodium (I)), ou le Rh-Ferrotane (tétrafluoroborate de 1,1'-bis[(2S,4S)-2,4-diéthylphosphétano]ferrocène-(1,5-cyclooctadiène)-rhodium (I)).

27. Procédé selon la revendication 1 ou la revendication 21, dans lequel l'hydrogénation de l'acide (IV, R² représente OH) est mise en oeuvre en présence d'une base.

28. Procédé selon la revendication 27, dans lequel la base est le bicarbonate de sodium, la cyclohexylamine, l'isopropylamine, la t-butylamine, l'adamantanamine ou la (S)-α-méthylbenzylamine.

29. Procédé selon la revendication 1 ou la revendication 21, dans lequel l'hydrogénation peut être mise en oeuvre sur un sel préformé (IV, R² représente O⁻ M⁺).

30. Procédé selon la revendication 1 ou la revendication 21, dans lequel l'hydrogénation est mise en oeuvre dans un solvant inerte, sous une pression positive d'hydrogène.

31. Procédé selon la revendication 30, dans lequel le solvant est un alcool aqueux en C₁₋₃ ou un alcool en C₁₋₃.

32. Procédé selon la revendication 1 ou la revendication 21, dans lequel la réaction est mise en oeuvre avec un catalyseur à base de ruthénium et la température de réaction est d'approximativement 60°C.

33. Procédé selon la revendication 1 ou la revendication 21, dans lequel la réaction est mise en oeuvre avec un catalyseur à base de rhodium et la température de réaction est d'approximativement 20°C.
